# EUROPÄISCHE PATENTSCHRIFT

(11) **EP 1 216 043 B1**
(45) Veröffentlichungstag und Bekanntmachung des Hinweises auf die Patenterteilung: **07.05.2003**
(21) Anmeldenummer: 00969359.9
(22) Anmeldetag: 29.09.2000
(51) Int. Cl.: A61K 31/385, A61K 9/20, A61K 7/00

(54) **ALPHA-LIPONSÄURE(-DERIVATE)ENTHALTENDE RETARDFORM**
RETARD FORM CONTAINING ALPHA-LIPOIC ACID (DERIVATIVES)
FORMULATION A EFFET RETARD CONTENANT DE L'ACIDE ALPHA-LIPOIQUE OU DES DERIVES DE CELUI-CI

(30) Priorität: 01.10.1999 DE 19947330; 16.09.2000 DE 10045904
(43) Veröffentlichungstag der Anmeldung: 26.06.2002
(73) Patentinhaber: Degussa AG, 83308 Trostberg (DE)
(72) Erfinder: SCHUHBAUER, Hans, 83308 Trostberg (DE); PISCHEL, Ivo, 83308 Trostberg (DE); BERNKOP-SCHNÜRCH, Andreas, A-1090 Wien (AT)
(74) Vertreter: Weiss, Wolfgang, Dipl.-Chem. Dr.
(86) Internationale Anmeldenummer: EP0009585
(87) Internationale Veröffentlichungsnummer: WO01024795

(56) Entgegenhaltungen:
- EP-A- 0 427 246
- EP-A- 0 560 092
- EP-A- 0 702 953

## Beschreibung

Gegenstand der vorliegenden Erfindung ist eine α-Liponsäure (-Derivate) enthaltende Retardform und deren Verwendung.

α-Liponsäure (Thioctsäure, 1,2-Dithiolan-3-pentansäure) kommtin geringen Konzentrationen in Form ihres R-Enantiomeren in pflanzlichen und tierischen Zellen als Naturstoff vor. Die ursprünglich als Wuchsfaktor entdeckte α-Liponsäure wirkt physiologisch in hydrophilen und lipophilen Medien als Coenzym bei der oxidativen Decarboxylierung von α-Ketocarbonsäuren wie z.B. Pyruvaten und als Antioxidans. Außerdem dient die α-Liponsäure der Regeneration von Vitamin C, Vitamin E, Glutathion und Coenzym Q10.

Die Synthesen von roher racemischer α-Liponsäure, von enantiomerenreiner R- oder S-α-Liponsäure, von Dihydroliponsäure oder deren Salze erfolgen auf bekannte oder analoge Weise, wie sie beispielsweise in Crévisy et al., Eur. J. Org. Chem. 1998, 1949, Fadnavis et al., Tetrahedron Asym. 1998, 9, 4109, Dhar et al., J. Org. Chem. 1992, 57, 1699, Adger et al., J. Chem. Soc. Chem. Commun. 1995, 1563, Dasaradhi et al., J. Chem. Soc. Chem. Commun. 1990, 729, Gopalan et al., J. Chem. Soc. Perkin Trans. I 1990, 1897, Yadav et al., J. Sci. Ind. Res. 1990, 49, 400, Tolstikov et. al., Bioorg. Khim. 1990, 16, 1670, Gopalan et al., Tetrahedron Lett. 1989, 5705, beschrieben oder zusammengefasst sind.

Als übliche Reinigungsmethode für rohe α-Liponsäure wird eine Umkristallisation aus Lösemitteln (z.B. aus n-Pentan, Cyclohexan, Methylcyclohexan, Ethylacetat) oder Gemischen von Lösemitteln (z.B. aus Ethylacetat und Hexan) verwendet, wie sie beispielsweise in Brookes et al., J. Chem. Soc. Perkin Trans. I 1988, 9, Segre et al., J. Am. Chem. Soc. 1957, 3503, Walton et al., J. Am. Chem. Soc. 1955, 77, 5144, Acker et al., J. Am. Chem. Soc. 1954, 76, 6483, beschrieben ist. Die auskristallisierte α-Liponsäure wird dann abfiltriert oder abzentrifugiert und anschließend mit gängigen Methoden getrocknet. Die so erhaltene kristalline α-Liponsäure wird schließlich zum fertigen Wirkstoff weiterverarbeitet.

Racemische α-Liponsäure wird bereits seit vielen Jahren zur Behandlung von Lebererkrankungen, Parästhesien und Neuropathien (z.B. autonome und periphere diabetische Polyneuropathie) eingesetzt; außerdem wurde ihr Einsatz als effektiver Inhibitor der Replikation von HIV-1-Viren diskutiert (vgl. Klin. Wochenschr.1991, 69(15), 722-724). Das Racemat der α-Liponsäure weist auch zytoprotektive, antiphlogistische und antinociceptive (analgetische) Eigenschaften auf. Darüberhinaus ist α-Liponsäure ein auch in lipophilen Medien gut löslicher Radikalfänger. Nachdem α-Liponsäure auch nachweislich den Glucose-Transport in Muskel- und Fettzellen stimuliert (vgl. Lipoic Acid in Health and Disease, Marcel Dekker Inc., New York 1997, S. 87ff.), ist überdies der Einsatz dieses Wirkstoffs zur Behandlung von Erkrankungen im Zusammenhang mit Typ 2 Diabetes möglich.

Klinische Studien zur Pharmakokinetik von α-Liponsäure haben jedoch sowohl eine nur sehr geringe absolute Bioverfügbarkeit von 24.1 - 38.2 % für das (R)-Enantiomer, und 19.1 - 28.3 % für das (S)-Enantiomer von α-Liponsäure gezeigt. Überdies wurde eine relativ kurze Plasma-Halbwertszeit nach peroraler Verabreichung von weniger als zwei Stunden beobachtet (Tabelle 1).

**Tabelle 1**

| Pharmakokinetische Parameter von α-Liponsäure-Enantiomeren nach einfacher peroraler Gabe verschiedener Darreichungsformen (nach Hermann und Niebch, Lipoic Acid in Health and Disease, Marcel Dekker, New York 1997, S. 346) | | | | | | | |
|---|---|---|---|---|---|---|---|
| 200 mg (±)-Liponsäure | | als Lösung, peroral | | als 4x50 mg-Tabletten | | als 200 mg-Tablette | |
| Enantiomer | | R | S | R | S | R | S |
| F⁽¹⁾ [%] | MW⁽²⁾ | 38.2 | 28.3 | 25.9 | 20.9 | 24.1 | 19.1 |
| | σ⁽³⁾ | ±15.2 | ±14.4 | ±17.1 | ±16.6 | ±12.7 | ±12.8 |
| Cₘₐₓ [µg ml⁻¹] | MW⁽²⁾ | 2.24 | 1.32 | 0.60 | 0.38 | 0.49 | 0.31 |
| | σ⁽³⁾ | ±1.21 | ±0.69 | ±0.41 | ±0.28 | ±0.27 | ±0.16 |
| tₘₐₓ [h] | MW⁽²⁾ | 0.21 | 0.21 | 0.70 | 0.70 | 0.90 | 0.90 |
| | σ⁽³⁾ | ±0.07 | ±0.07 | ±0.41 | ±0.41 | ±0.74 | ±0.74 |
| t_{1/2} [h] | MW⁽²⁾ | 0.24 | 0.15 | 0.71 | 0.82 | 0.33 | 0.33 |
| | σ⁽³⁾ | ±0.29 | ±0.08 | ±0.68 | ±0.99 | ±0.20 | ±0.24 |

| | | | | | | | |
|---|---|---|---|---|---|---|---|
| ⁽¹⁾ F: Bioverfügbarkeit ⁽²⁾ arithmetischer Mittelwert ⁽³⁾ Standardabweichung | | | | | | | |

Diese Nachteile von unbefriedigender Bioverfügbarkeit und geringer Plasmahalbwertszeit hat man deshalb versucht, mit Hilfe von so genannten Retardformen, die eine verzögerte Freisetzung gewährleisten sollen, zu überwinden.

So ist beispielsweise aus der DE-OS 44 13 350 eine feste, pelletförmige Retardform bekannt, die neben einer biologisch wirksamen Verbindung a) ("Wirkstoff") b) noch mindestens ein in Wasser und in gastrointestinalen Flüssigkeiten unlösliches natürliches, halbsynthetisches oder synthetisches Polymer, c) mindestens eine wasserunlösliche lipophile Komponente mit Weichmachereigenschaften bezüglich des Polymeren b) und Gleit- bzw. Schmiermitteleigenschaften, d) mindestens ein in Wasser oder gastrointestinalen Flüssigkeiten kolloidal lösliches, hochviskose Lösungen oder Gele bildendes oder mindestens quellbares natürliches oder halbsynthetisches hydrophiles Polymer ("Gelbildner") enthält, sowie fakultativ ein oder mehrere der üblichen Formulierungshilfsstoffe, wobei als Gelbildner wasserunlösliche Chitin-Derivate wie Chitosan genannt sind. Der Gelbildner soll daher insbesondere das Herausdiffundieren des Wirkstoffes aus dem Inneren der Pellets ermöglichen. Als ein möglicher Wirkstoff ist u. a. die Thioctsäure (α-Liponsäure) genannt.

Bei dieser sehr komplex zusammengesetzten Retardform handelt es sich um Pellets, die durch Schmelzextrusion bei Temperaturen zwischen 50 und 200 °C gewonnen werden, wobei der so genannte Heißabschlag bevorzugt wird.

Als nachteilig bei dieser Retardform muss - neben ihrer mehrkomponentigen Polymerzusammensetzung - vor allem im Hinblick auf die α-Liponsäure als Wirkstoff das Extrusionsverfahren angesehen werden:

α-Liponsäure ist eine bekanntermaßen wärmelabile Verbindung, weshalb sowohl die für den Extrusionsprozess vorgesehene Temperatur von 50 bis 200 °C als auch der ebenfalls bevorzugte Heißabschlag zwar weniger die verwendeten Polymere, sehr wohl aber den möglichen Wirkstoff α-Liponsäure negativ beeinflussen, weshalb gerade im Fall der α-Liponsäure von einer gravierenden Wärmedestruktion auszugehen ist.

Die Kombination eines hydrophilen und amidhaltigen Polymers mit einer endogenen Verbindung in einer medizinischen Zusammensetzung zur Ausbildung einer topischen Barriereformulierung ist aus der WO 98/26788 bekannt. Als geeignetes Polymer ist u. a. eines aus der Gruppe der nativen Chitosane oder katonische Derivate davon genannt. Das Polymer muss an eine anionische Scavenger-Substanz gebunden sein, u. a. in Form der genannten endogenen Verbindung, die zudem eine Amino- und/oder Thiolfunktion aufweisen muss. Hauptanwendungszweck dieser Formulierung sind Hauterkrankungen mit allergischem Hintergrund.

Eine Formulierung zur kontrollierten Freisetzung von α-Liponsäure ist auch aus der WO 99/61004 bekannt, gemäß der eine therapeutisch wirksame Menge α-Liponsäure und ein bindendes Material so kombiniert werden, dass die Liponsäure im Gastrointestinaltrakt vor einem chemischen Abbau geschützt und gleichzeitig die kontrollierte Freisetzung der Liponsäure gewährleistet wird. Als bindendes Material wird eine wässrige Lösung des Celluloseacetatphthalates und mikrokristalline Cellulose verwendet. In den dazu angeführten Beispielen wird zwar die antidiabetische Wirkung dieser Formulierung über den gemessenen Blutglucosespiegel aufgezeigt, die behauptete Retardwirkung auf die α-Liponsäure ist allerdings nicht belegt.

Aus dem bekannten Stand der Technik heraus und insbesondere auf Grund der damit verbundenen Nachteile hat sich somit für die vorliegende Erfindung die Aufgabe gestellt, eine α-Liponsäure(-Derivate) enthaltende Retardform zu entwickeln, die eine Verbesserung der Bioverfügbarkeit der α-Liponsäure und/oder deren geeigneter Derivate ermöglicht und die einen über mehrere Stunden konstant bleibenden α-Liponsäure-Plasmaspiegel gewährleistet, um so den therapeutischen Effekt von α-Liponsäure (-Derivaten) deutlich verbessern zu können. Mit der neuen Retardform sollte außerdem einerseits die Resorption von α-Liponsäure oder deren geeigneter Derivate davon z.B. aus dem Gastrointestinal-(GI)-Trakt verbessert sowie andererseits eine kontrollierte Wirkstofffreigabe über etwa acht Stunden hinaus gewährleistet werden.

Gelöst wurde diese Aufgabe mit einer Retardform, die (a) ein oder mehrere kationogene Polymere, (b) α-Liponsäure oder/und mindestens eines ihrer Derivate und (c) mindestens eine von (b) verschiedenen Säure enthält, wobei als Komponenten (a) und (b) günstigerweise physiologisch und pharmakologisch verträgliche Substanzen eingesetzt werden. Der pH-Wert der Gesamtformulierung beträgt vorzugsweise 3,0 bis 8,5, besonders bevorzugt 4,0 bis 7,0. Überraschend wurde gefunden, dass neben der gewünschten kontrollierten Wirkstofffreigabe für mehr als acht Stunden und der verlängerten Gl-Transitzeit auch eine beschleunigte Penetration der Inhaltsstoffe stattfindet. Völlig unerwartet aber ist mit der erfindungsgemäßen Retardform eine zum Teil drastisch erhöhte Bioverfügbarkeit von α-Liponsäure und deren Derivate verbunden.

Die vorliegende Erfindung stellt somit eine Darreichungsform dar, mit der durch Kombination eines anionogenen Wirkstoffes wie α-Liponsäure mit einer speziellen kationogenen Trägermatrix Formulierungen zur Verfügung gestellt werden, die auf Grund von überwiegend ionischen Wechselwirkungen zwischen den beiden Hauptkomponenten den Wirkstoff mit zeitlicher Verzögerung freigeben.

Für die erfindungsgemäßen Retardformen haben sich insbesondere sowohl racemische als auch enantiomerenreine R-(+)-α-Liponsäure oder S-(-)-α-Liponsäure bzw. beliebige Mischungen davon bewährt. Ebenso möglich ist es, racemische Dihydroliponsäure (6,8-Dimercaptooctansäure) oder enantiomerenreine S-(+)-Dihydroliponsäure oder R-(-)-Dihydroliponsäure bzw. beliebige Mischungen davon einzusetzen. Beispiele für weitere Liponsäurederivate sind die (auch unter dem Namen "β-Liponsäure" literaturbekannten) Sulfoxide 1,2-Dithiolan-1-oxid-3-valeriansäure und 1,2-Dithiolan-2-oxid-3-valeriansäure, jeweils in enantiomerenreiner Form oder in Form beliebiger Mischungen oder Racemate einzelner oder sämtlicher Regioisomere und/oder Diastereomere. Desweiteren ist auch racemisches Liponsäureamid (Thioctsäureamid) oder enantiomerenreines S-Liponsäureamid oder R-Liponsäureamid bzw. beliebige Mischungen davon geeignet.

In einer weiteren bevorzugten Ausführungsform der Erfindung wird die α-Liponsäure oder Dihydroliponsäure ganz oder teilweise in Form ihrer Salze als anionogene Komponente gemeinsam mit einem kationogenen Polymeren und der Säurekomponente (c) eingesetzt. So sind Salze besonders geeignet, die Kationen aus der Reihe der Alkali- (wie z.B. Natrium oder Kalium) oder Erdalkalimetalle (wie z.B. Calcium oder Magnesium) enthalten. Es kann jedoch auch ohne weiteres auf andere Salze der α-Liponsäure zurückgegriffen werden, wobei dann deren Kationen insbesondere aus der Reihe Eisen, Kupfer, Zink, Palladium, Vanadium und Selen stammen.

Auch α-Liponsäure-Salze, die organische Kationen und hier vorzugsweise offenkettige oder cyclische Ammoniumverbindungen, wie Benzylammonium, Diisopropylammonium, Triethylammonium oder Cyclohexylammonium, sowie komplexe Kationen ggf. mit metallischen Zentralatomen wie z.B. Eisen (III), Chrom(III) oder Cobalt(II) und neutralen, kationischen oder anionischen Liganden wie z.B. Wasser, Ammoniak, Carbonyl, Cyano oder Nitroso, oder Oxokationen, wie Oxovanadium(V) (VO₂⁺) oder Oxovanadium (IV) (VO²⁺)) enthalten, sind für die Retardformen gemäß vorliegender Erfindung bestens geeignet.

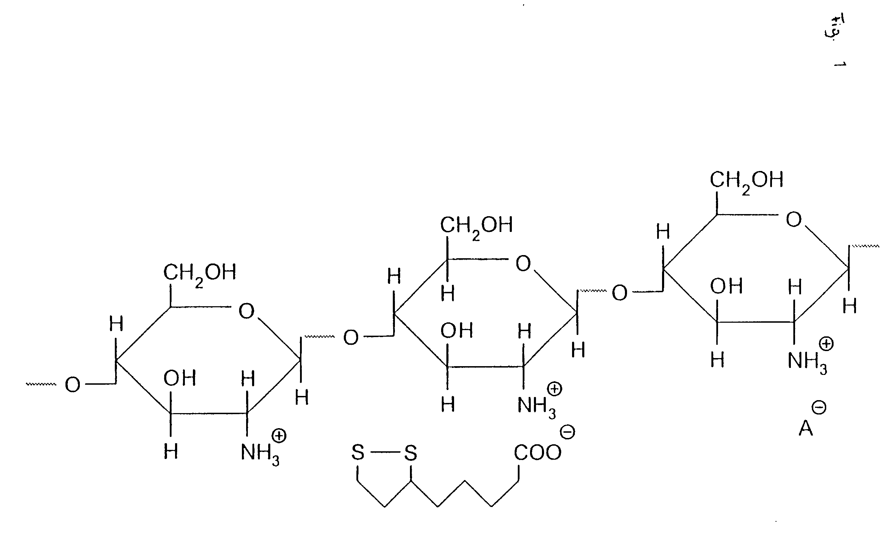

Die bereits erwähnten ionischen Wechselwirkungen zwischen dem kationogenen Polymeren (a) und der α-Liponsäure bzw. deren Derivaten mit anionogenem Charakter auf der einen Seite sowie der Säure-Komponente (c) auf der anderen Seite werden erfindungsgemäß vorzugsweise durch die Verwendung des Polymers Chitosan (Poly-D-Glucosamin) oder eines Chitosan-Salzes (wie z. B. Chitosan-Hydrochlorid, -Acetat oder -Glutamat), oder durch die Verwendung von Poly-L-Lysin, basischen Lektinen (Glycoproteinen, z. B. aus Extrakten wie Phytohämagglutininen), oder anderen basischen Polypeptiden, Polysacchariden (wie z. B. Hexosaminzucker) oder Biopolymeren pflanzlichen, tierischen oder synthetischen Ursprungs sowie beliebigen Mischungen daraus erzielt. Dabei lässt sich dieser Mechanismus einer retardierenden Wirkstoff-Adhäsion auf Basis von ionischen, dipolaren sowie anderen intermolekularen Wechselwirkungen prinzipiell wie in Abbildung 1 gezeigt, darstellen und erklären.

Das als kationogenes Polymer bevorzugte Chitosan kann durch chemische Umwandlung (Deacetylierung) aus Chitin (Poly-N-Acetyl-D-Glucosamin) gewonnen werden. Zu den natürlichen Quellen von Chitosan gehören Krill sowie die Schalen von Krabben, Langusten, Hummer und anderen Vertreter der Crustaceen. Hochmolekulares Chitosan mit einer Molmasse von 500.000 bis 600.000 Dalton und einem Deacetylierungsgrad von 80 - 95 % ist besonders geeignet für den Einsatz in kosmetischen Formulierungen sowie in Nahrungsergänzungsmittein.

Der Einsatz von Chitosan als Pharmazeutikum beispielsweise als Antikrebsmittel, zur Wundheilung, bei Arthritis sowie bei gastrointestinalen Erkrankungen und als Saatgutschutz in der Landwirtschaft ist bekannt.

Der Gehalt an α-Liponsäure-Komponente (b) in der Retardform kann in weiten Grenzen variiert werden. Es hat sich jedoch als besonders vorteilhaft erwiesen, den Gewichtsanteil der α-Liponsäure-Komponente bezogen auf das Gesamtgewicht der Retardform zwischen 0,1 und 99 %, insbesondere zwischen 20 und 90%, einzustellen. Analog dazu sollte der Gewichtsanteil an kationogenen Polymeren vorzugsweise zwischen 0,1 und 90 % und insbesondere zwischen 5 und 50 % eingestellt werden.

Auch die Anteile der Säurekomponente (c) können breit streuen: So sind gemäß Erfindung vorzugsweise Anteile von 0,001 bis 80 Gew.-% vorgesehen, wobei allerdings Anteile von 0,1 bis 50 Gew.-% und besonders Anteile von 0,1 bis 25 Gew.-% zu bevorzugen sind.

Dieses breite Anteilsspektrum hängt nicht zuletzt mit der Vielzahl an möglichen Säuren zusammen, die gemäß vorliegender Erfindung als Komponente (c) in Frage kommen: So können organische oder anorganische Brönstedsäuren, wie z. B. Essigsäure, Salzsäure und Glutaminsäure, ebenso eingesetzt werden wie organische oder anorganische Lewissäuren, aus deren Reihe sich vor allem Kohlendioxid, Ca²⁺ und Fe²⁺ besonders eignen.

In Frage kommen aber auch komplexe Säuren, insbesondere Hexaaquoaluminium-(III) [Al(H₂O)₆³⁺] oder Hexacyanoeisen-(II)-säure [H₄(Fe(CN)₆)], aber auch polymere Säuren, von denen Polyphosphorsäure (PPA), eine Isopolysäure, wie z. B. Heptamolybdänsäure (H₆M₇O₂₄), oder eine Heteropolysäure, wie z. B. Dodecawolframphosphorsäure (H₃[PW₁₂O₄₀]), besonders zu bevorzugen sind.

Schließlich ist es in diesem Zusammenhang auch möglich, beliebige Mischungen der einzelnen Säureformen untereinander aber auch zwischen den einzelnen Säureformen heranzuziehen.

Im Rahmen der Erfindung ist auch der Einsatz üblicher Formulierungs-Hilfsmittel vorgesehen, die dann allerdings nur als zusätzliche fakultative Komponente anzusehen sind. In Frage kommen hierbei insbesondere Füllstoffe, Schmiermittel, Fließhilfsmittel, Formentrennmittel, Weichmacher, Treibmittel, Stabilisatoren, Farbstoffe, Streckmittel, Bindemittel, Sprengmittel, Netzmittel, Fließmittel oder Gegenklebemittel.

Aus dem breiten Spektrum der möglichen geeigneten Formulierungs-Hilfsmittel kommen als Füllstoffe Oxide von Magnesium, Aluminium, Silizium oder Titan, mikrokristalline Cellulose und Cellulosepulver, Stärken und deren Derivate (beispielsweise Maltodextrine), Lactose, Mannit und Calciumdiphosphat, als Schmiermittel Stearate von Aluminium und Calcium, Talkum oder Silicone, als Fließhilfsmittel Magnesiumstearat, kolloidales Siliciumdioxid, Talkum oder Aerosil, als Weichmacher niedermolekulare Polyalkylenoxide, niedermolekulare organische Weichmacher wie Glycerin, Pentaerythrit, Glycerin-Monoacetat, -Diacetat oder -Triacetat, Propylenglycol, Sorbit oder Na-Diethylsulfonsuccinat, als Farbstoffe Azofarbstoffe, (an-)organische Pigmente oder natürliche Farbmittel, oder sonstige übliche Hilfsstoffe wie Zucker(-alkohole), Polymere, Phosphate und Tenside in Frage, die im Bedarfsfall jeweils bevorzugt in Konzentrationen zwischen 0,02 und 50 Gew.-% bezüglich des Gesamtgewichts der Retardform enthalten sein sollten.

Schließlich sieht die vorliegende Erfindung neben speziellen Retardzusammensetzungen auch bevorzugte Retardformen vor, die nach einem bestimmten Verfahren hergestellt werden:

Für die erfindungsgemäße Retardform kann beispielsweise kommerziell verfügbares Chitosan, wie es üblicherweise aus Krabbenschalen gewonnen wird, zunächst in saurer wäßriger Lösung gequollen und anschließend mit kristalliner α-Liponsäure homogenisiert und nach Zusatz der Säure feuchtgranuliert werden. Aus dem getrockneten Granulat werden dann Tabletten nach konventionellen Methoden gepresst. Der Gewichtsanteil von α-Liponsäure in derartigen Tabletten kann dabei über 75% betragen.

Vorzuziehen ist hierbei allerdings gemäßErfindung eine Vorgehensweise, bei der
1) die Komponente (a) mit der Komponente (c) vorzugsweise im Gewichtsverhältnis 1 : 2 bis 1 : 4 versetzt wird, dann diese Mischung mit Wasser versetzt wird und die entstandene Mischung, z.B. als Lösung mit der α-Liponsäure-Komponente (b) im bevorzugten Gewichtsverhältnis Mischung: Komponente (b) 1 : 0,3 - 0,003 homogenisiert wird,
2) das Homogenat aus 1) einer Feuchtgranulation unterzogen wird und die Granulate vorzugsweise bei Temperaturen zwischen 5 und 50 °C, besonders bevorzugt zwischen 25 und 40°C getrocknet werden, sowie
3) die Trockengranulate tablettiert werden.

Die mit Chitosan oder einem gemäß Erfindung anderen geeigneten kationogenen Polymer sowie der Säure-Komponente (c) homogenisierte, feuchtgranulierte und tablettierte α-Liponsäure bzw. deren Derivate können aber auch mittels eines beliebigen anderen Verfahrens hergestellt werden. Es spielt hierbei nämlich vorrangig keine Rolle, ob die α-Liponsäure (-Derivate) beispielsweise durch Umkristallisation mit einem organischen Lösemittel oder Lösemittelgemisch hergestellt wurden oder ob die rohe α-Liponsäure ohne jegliches organisches Lösemittel eingesetzt wird.

Auf Grund der günstigen Eigenschaften der erfindungsgemäßen Retardform wird deren Verwendung als Nahrungsergänzungsmittel genauso vorzugsweise beansprucht wie die Verwendung als Arzneimittel und/oder Kosmetikum, wobei die Retardform für perorale, dermale, parenterale, rektale, vaginale oder lokale (topische) Applikationen eingesetzt werden kann.

Außerdem ist im Rahmen der vorliegenden Erfindung vorgesehen, die beanspruchte Retardform als Gele, halbfeste Darreichungsformen oder feste Lösungen zu verwenden oder auch als Grundlage zur Herstellung von Gelen, festen Lösungen und insbesondere halbfesten Darreichungsformen.

Die nachfolgenden Abbildungen und Beispiele belegen die Vorteile der erfindungsgemäßen Retardform. Es zeigen
- Abb. 1: Wechselwirkungen zwischen kationogenem Chitosan (als Beispiel für Komponente a)), anionogener α-Liponsäure (Komponente b)) sowie einer weiteren Säure-Komponente (c) (als Anion A^{⊖} dargestellt);
- Abb. 2: den Vergleich der Einflüsse von Chitosan und Essigsäure auf die Retardierung von α-Liponsäure. Freisetzungsstudien von α-Liponsäure wurden angestellt (i) ohne Chitosan und Essigsäure (■---■), (ii) mit Chitosan ohne Essigsäure (○---○), (iii) mit Chitosan-Essigsäure 4:1 (m/m) (Δ---Δ), (iv) mit Chitosan-Essigsäure 2:1 (m/m) (X---X), (v) mit Chitosan-Essigsäure 1:1 (m/m) (●---●)
und
- Abb. 3: das Dissolutionsprofil von mit Essigsäure hergestellen α-Liponsäure-Chitosan-Tabletten (400mg) in 600 ml demineralisiertem Wasser bei 37 °C. (α-Liponsäure-Anteil >75%)

### Beispiele

### 1. Diffusionsstudie

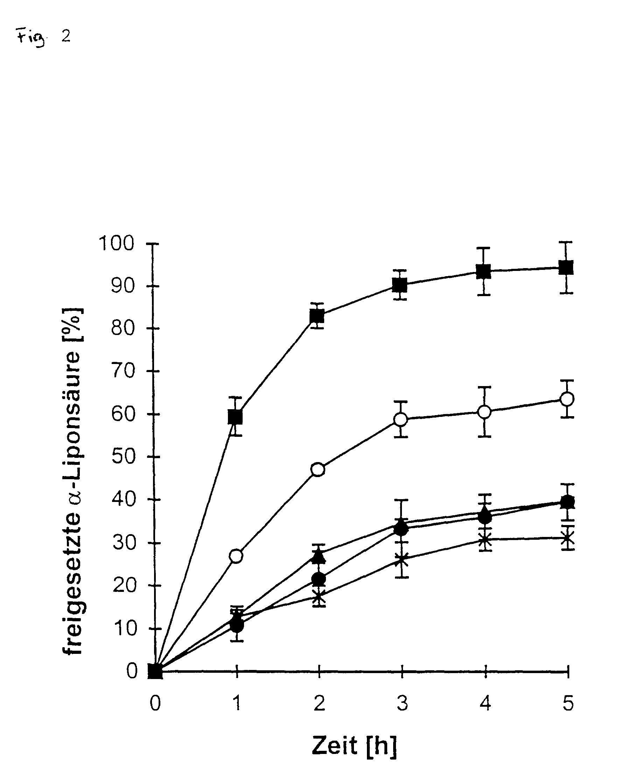

Um über einen Zeitraum von 24 Stunden die verzögerte Freisetzung von α-Liponsäure aus der Darreichungsform zu gewährleisten, wurde Chitosan als polymere Wirkstoffträgermatrix eingesetzt. Auf Grund ionischer Wechselwirkungen dieses kationogenen Polymeren mit dem anionogenen Wirkstoff α-Liponsäure wird diese kontinuierlich freigesetzt. Bei der vorliegenden Diffusionsstudie wurde der Einfluss von Chitosan auf das Diffusionsverhalten von α-Liponsäure untersucht. Die Ergebnisse dieser Untersuchung sind in Fig. 2 dargestellt und veranschaulichen den starken Einfluss des kationogenen Polymeren auf das Diffusionsverhalten des Wirkstoffes. Während ohne Chitosan das Konzentrationsgleichgewicht der α-Liponsäure innerhalb und außerhalb des Dialysegefäßes innerhalb von ca. 5 Stunden erreicht werden konnte, wurden lediglich 63,8 % ± 4,3 % dieses Gleichgewichts in Gegenwart des kationogenen Polymers Chitosan erreicht. Auf der einen Seite kann Chitosan in wässrigen Lösungen nur in ionischer Form hydriert werden, auf der anderen Seite haben vorangehende Studien eindeutig gezeigt, dass α-Liponsäure als Gegenion zu hydrophob ist, um ein ausreichendes Quellen des Polymers zu bewirken. Wie die vorliegende Diffusionsstudie zeigt, ist die Zugabe einer eher polaren Säure zusätzlich zum Wirkstoff notwendig, um die Hydratation des Polymeren zu gewährleisten.

Auf Grund ihres vergleichsweise hohen pKa-Wertes von ca. 4,76, der eine ionische Bindung des Wirkstoffes zulässt, keine toxischen Risiken aufwirft und eine exzellente Hydratation des Chitosan gewährleistet, wurde Essigsäure ausgewählt.

Wie die Ergebnisse der Diffusionsstudie zeigen, bewirken schon geringe Konzentrationen an Essigsäure einen gesteigerten Retardeinfluss des Chitosan auf den Wirkstoff α-Liponsäure: Allein die Belegung jeder vierten Aminogruppe des Chitosans mit Essigsäure (Chitosan-1/4-Acetat) führte zu einer signifikanten Verringerung der Freisetzungsrate von α-Liponsäure aus dem Polymeren. Ein Grund für diese Beobachtung kann darin gesehen werden, dass im Chitosan freie primäre Aminogruppen zunehmen, die für den Wirkstoff zugänglich sind, was auf den höheren Hydratationsgrad, verursacht durch die Essigsäure, zurückgeführt werden kann.

Sobald eine Essigsäure-Konzentration erreicht wird, die sämtliche primären Aminogruppen des Polymers für den Wirkstoff zugänglich machen, kann der Retard-Effekt des Polymeren nicht mehr gesteigert werden.

Andererseits scheint die weitere Zugabe von Essigsäure den Retard-Effekt zu verringern, da dieser bei einem Verhältnis Chitosan/Acetat = 1 : 1 mit 39,8 ± 0,9 % innerhalb von 5 Stunden signifikant geringer war als bei einem Verhältnis Chitosan zu Acetat = 1 : 2 (31,4 ± 2,8 %).

Diese Beobachtung kann mit einem Konkurrenzverhalten des Wirkstoffes α-Liponsäure und der Essigsäure um die frei zugänglichen Aminogruppen des Polymers erklärt werden. Schließlich ist zu beachten, dass mit zunehmender Essigsäurezugabe auch größere Mengen des Wirkstoffes vom Polymer entfernt werden.

### 2. Freisetzungsstudie

Untersuchungen zum Freisetzungsprofil der Tabletten wurden nach international anerkannten Vorschriften durchgeführt, wie sie beispielsweise im Europäischen Arzneibuch zu finden sind.

### Beispiel 1

5 g Chitosan aus Krabbenschalen mit einem Deacetylierungsgrad von über 85% wurden in 10 ml Eisessig und 65 ml demineralisiertem Wasser 24 Stunden bei Raumtemperatur gequollen. Dieser Ansatz wurde in der Folge mit 24 g α-Liponsäure homogenisiert und feuchtgranuliert. Anschließend wurden aus dem bei 40°C getrockneten Granulat Tabletten von 10 mm Durchmesser und 400 mg Gewicht gepresst (Korsch, Typ EKO-DMS, Berlin, Deutschland). Der Anteil an α-Liponsäure in diesen Tabletten betrug über 75% (m/m).

### Beispiel 2

50 g Chitosan aus Krabbenschalen mit einem Deacetylierungsgrad von über 85% wurden in 100 ml Eisessig und 750 ml demineralisiertem Wasser 24 Stunden bei Raumtemperatur gequollen. Dieser Ansatz wurde in der Folge mit 50 g α-Liponsäure homogenisiert und feuchtgranuliert. Anschließend wurden aus dem bei 40°C getrockneten Granulat Tabletten von 10 mm Durchmesser und 400 mg Gewicht gepresst (Korsch, Typ EKO-DMS, Berlin, Deutschland). Der Anteil an α-Liponsäure in diesen Tabletten betrug dabei etwa 50% (m/m).

### Versuchsergebnis

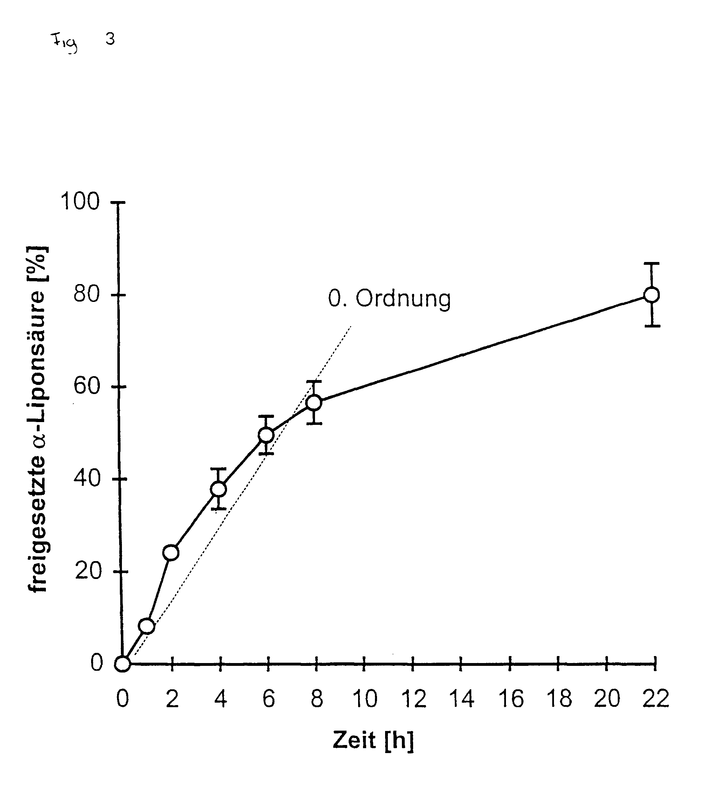

Freisetzungsversuche mit diesen Tabletten zeigten eine starke Retardierung durch den kombinierten Einsatz von α-Liponsäure mit Chitosan. Das Dissolutionsprofil der α-Liponsäure-Chitosan-Tabletten (400 mg) in 600 ml demineralisiertem Wasser bei 37 °C ist in Abb. 3 dargestellt. Die gezeigten Werte sind Mittelwerte aus drei Freisetzungsstudien mit der entsprechenden Standardabweichung. innerhalb der ersten 8 Stunden entspricht diese Freisetzung annähernd einer der 0. Ordnung. Die gezeigte Retardierung, bei der nach 22 Stunden erst 80% an α-Liponsäure freigesetzt werden, wurde gewählt, da zum einen diese Freisetzung in vivo durch eine hohe Elektrolytkonzentration beschleunigt wird und zum anderen eine Teilresorption von α-Liponsäure selbst noch im Kolon erfolgt.

## Patentansprüche

1. Retardform, enthaltend
(a) ein oder mehrere kationogene Polymere,
(b) α-Liponsäure oder/und ein Derivat davon und
(c) mindestens eine von (b) verschiedene Säure.

2. Retardform nach Anspruch 1,
**dadurch gekennzeichnet,**
**dass** die Komponente (b) eine racemische α-Liponsäure, eine enantiomerenreine R-(+)- oder S-(-)-α-Liponsäure oder Mischungen daraus enthält.

3. Retardform nach Anspruch 1,
**dadurch gekennzeichnet,**
**dass** die Komponente (b) eine racemische Dihydroliponsäure, eine enantiomerenreine (+)-Dihydroliponsäure oder (-)-Dihydroliponsäure oder Mischungen daraus enthält.

4. Retardform nach einem der Ansprüche 1 bis 3,
**dadurch gekennzeichnet,**
**dass** die α-Liponsäure oder Dihydroliponsäure ganz oder teilweise in Form ihrer Salze vorliegt.

5. Retardform nach Anspruch 4,
**dadurch gekennzeichnet,**
**dass** die Salze der α-Liponsäure oder Dihydroliponsäure Kationen aus der Gruppe der Alkali- oder Erdalkalimetalle enthalten.

6. Retardform nach Anspruch 4,
**dadurch gekennzeichnet,**
**dass** die Salze der α-Liponsäure oder Dihydroliponsäure Kationen aus der Gruppe Eisen, Kupfer, Zink, Palladium, Vanadium und Selen enthalten.

7. Retardform nach Anspruch 4,
**dadurch gekennzeichnet,**
**dass** die Salze der α-Liponsäure oder Dihydroliponsäure organische Kationen, insbesondere offenkettige oder cyclische Ammoniumverbindungen wie Benzylammonium, Diisopropylammonium, Triethylammonium, Cyclohexylammonium, komplexe Kationen ggf. mit metallischen Zentralatomen wie z. B. Eisen(III), Chrom(III) oder Cobalt(II) und neutralen, kationischen oder anionischen Liganden wie z. B. Wasser, Ammoniak, Carbonyl, Cyano oder Nitroso, oder Oxokationen wie Oxovanadium(V) (VO₂⁺) oder Oxovanadium (IV) (VO²⁺) enthalten.

8. Retardform nach einem der Ansprüche 1 bis 7,
**dadurch gekennzeichnet,**
**dass** die Komponente (a) ein kationogenes Polymer, ausgewählt aus Chitosan (Poly-D-Glucosamin) oder Chitosan-Salzen (wie z. B. Chitosan-Hydrochlorid, -Acetat, -Glutamat), Poly-L-Lysin, basischen Lektinen (Glycoproteine, z. B. aus Extrakten wie Phytohämagglutininen), oder anderen basischen Polypeptiden, Polysacchariden (wie z. B. Hexosaminzucker) oder Biopolymeren pflanzlichen, tierischen oder synthetischen Ursprungs sowie beliebigen Mischungen daraus enthält.

9. Retardform nach einem der Ansprüche 1 bis 8,
**dadurch gekennzeichnet,**
**dass** der Anteil an kationogenem Polymer 0,1 bis 90 Gew.-%, insbesondere 5 bis 50 Gew.-%, jeweils bezogen auf das Gewicht der Komponenten (a), (b) und (c) der Retardform beträgt.

10. Retardform nach einem der Ansprüche 1 bis 9,
**dadurch gekennzeichnet,**
**dass** die α-Liponsäure-Komponente in Anteilen von 0,1 bis 99 Gew.-%, insbesondere in Anteilen von 20 bis 90 Gew.-%, jeweils bezogen auf das Gewicht der Komponenten (a), (b) und (c) der Retardform vorliegt.

11. Retardform nach einem der Ansprüche 1 bis 10,
**dadurch gekennzeichnet,**
**dass** die Säure-Komponente (c) eine organische oder anorganische Brønstedt-Säure, insbesondere Essigsäure, Salzsäure oder Glutaminsäure enthält.

12. Retardform nach einem der Ansprüche 1 bis 10,
**dadurch gekennzeichnet,**
**dass** die Säure-Komponente (c) eine organische oder anorganische Lewis-Säure, insbesondere Kohlendioxid, Ca²⁺ oder Fe²⁺ enthält.

13. Retardform nach einem der Ansprüche 1 bis 10,
**dadurch gekennzeichnet,**
**dass** die Säure-Komponente (c) eine komplexe Säure, insbesondere Hexaaquoaluminium-(III)[(Al(H₂O)₆³⁺]oderHexacyanoeisen-(II)-säure [H₄(Fe(CN)₆)] enthält.

14. Retardform nach einem der Ansprüche 1 bis 10,
**dadurch gekennzeichnet,**
**dass** die Säure-Komponente (c) eine polymere Säure, insbesondere Polyphosphorsäure (PPA), eine Isopolysäure wie z. B. Heptamolybdänsäure (H₆M₇O₂₄), oder eine Heteropolysäure wie z. B. Dodecawolframphosphorsäure (H₃[PW₁₂O₄₀]) enthält.

15. Retardform nach einem der Ansprüche 1 bis 14,
**dadurch gekennzeichnet,**
**dass** die Säure-Komponente (c) in Anteilen von 0,001 bis 80 Gew.-%, insbesondere in Anteilen von 0,1 bis 50 Gew.-% und besonders bevorzugt in Anteilen von 1,0 bis 25 Gew.-%, jeweils bezogen auf das Gewicht der Komponenten (a), (b) und (c) der Retardform vorliegt.

16. Retardform nach einem der Ansprüche 1 bis 15,
**dadurch gekennzeichnet,**
**dass** sie zusätzlich Formulierungs-Hilfsmittel wie Füllstoffe, Schmiermittel, Fließhilfsmittel, Formentrennmittel, Weichmacher, Treibmittel, Stabilisatoren, Farbstoffe, Streckmittel, Bindemittel, Sprengmittel, Netzmittel, Fließmittel oder Gegenklebemittel enthält.

17. Retardform nach Anspruch 16,
**dadurch gekennzeichnet,**
**dass** sie als Füllstoffe anorganische Füllstoffe wie z. B. Oxide von Magnesium, Aluminium, Silizium oder Titan, mikrokristalline Cellulose und Cellulosepulver, Stärken und deren Derivate (beispielsweise Maltodextrine), Lactose, Mannit und Calciumdiphosphat, als Schmiermittel Stearate von Aluminium und Calcium, Talkum oder Silicone, als Fließhilfsmittel Magnesiumstearat, kolloidales Siliciumdioxid, Talkum oder Aerosil, als Weichmacher niedermolekulare Polyalkylenoxide, niedermolekulare organische Weichmacher wie Glycerin, Pentaerythrit, Glycerin-Monoacetat, -Diacetat oder -Triacetat, Propylenglycol, Sorbit oder Na-Diethylsulfonsuccinat, als Farbstoffe Azofarbstoffe, (an-)organische Pigmente oder natürliche Farbmittel, oder sonstige übliche Hilfsstoffe wie Zucker(-alkohole), Polymere, Phosphate und Tenside, bevorzugt in jeweiligen Anteilen zwischen 0,02 bis 50 Gew.- % bezogen auf das Gesamtgewicht enthält.

18. Retardform nach einem der Ansprüche 1 bis 17,
**dadurch gekennzeichnet,**
**dass** sie erhältlich ist, indem
1) die Komponente (a) mit der Komponente (c) vorzugsweise im Gewichstverhältnis 1 : 2 bis 1 : 4 versetzt wird, dann diese Mischung mit Wasser versetzt wird und die entstandene Mischung mit der α-Liponsäure-Komponente (b) im bevorzugten Gewichtsverhältnis Mischung : Komponente (b) 1 : 0,3 - 0,003 homogenisiert wird,
2) das Homogenat aus 1) einer Feuchtgranulation unterzogen wird und die Granulate bei Temperaturen zwischen 5 und 50 °C, vorzugsweise zwischen 25 und 40 °C getrocknet werden, sowie
3) die Trockengranulate tablettiert werden.

19. Verwendung der Retardform nach einem der Ansprüche 1 bis 18 zur Herstellung eines Nahrungsergänzungsmittels.

20. Verwendung der Retardform nach einem der Ansprüche 1 bis 18 zur Herstellung eines Arzneimittels.

21. Verwendung der Retardform nach einem der Ansprüche 1 bis 18 zur Herstellung eines Kosmetikums.

22. Verwendung nach einem der Ansprüche 19 bis 21 zur Herstellung peroraler, dermaler, parenteraler, vaginaler oder lokaler (topischer) Applikationsformen.

23. Verwendung nach einem der Ansprüche 19 bis 22 zur Herstellung von Gelen, halbfesten Darreichungsformen oder festen Lösungen oder als Grundlage davon.

24. Verwendung nach einem der Ansprüche 19 bis 23 zur Verbesserung der Resorption von α-Liponsäure und Derivaten davon.

25. Verwendung nach einem der Ansprüche 19 bis 24 zur Verlängerung der kontrollierten Wirkstoffabgabe auf eine Zeitdauer von über etwa 8 Stunden.

26. Verwendung nach einem der Ansprüche 19 bis 25 zur Erhöhung der Bioverfügbarkeit von α-Liponsäure oder/und deren Derivaten.

## Claims

1. Sustained release form comprising
(a) one or more cationic polymers,
(b) α-lipoic acid or/and a derivative thereof and
(c) at least one acid different from (b).

2. Sustained release form according to Claim 1, **characterized in that** component (b) comprises a racemic α-lipoic acid, an enantiopure R-(+)- or S-(-)-α-lipoic acid or mixtures thereof.

3. Sustained release form according to Claim 1, **characterized in that** component (b) comprises a racemic dihydrolipoic acid, an enantiopure (+)-dihydrolipoic acid or (-)-dihydrolipoic acid or mixtures thereof.

4. Sustained release form according to any of Claims 1 to 3, **characterized in that** the α-lipoic acid or dihydrolipoic acid is present in whole or in part in the form of the salts thereof.

5. Sustained release form according to Claim 4, **characterized in that** the salts of α-lipoic acid or dihydrolipoic acid comprise cations from the group of alkali metals or alkaline earth metals.

6. Sustained release form according to Claim 4, **characterized in that** the salts of α-lipoic acid or dihydrolipoic acid comprise cations from the group of iron, copper, zinc, palladium, vanadium and selenium.

7. A sustained release form according to Claim 4,
**characterized in that** the salts of α-lipoic acid or dihydrolipoic acid comprise organic cations, in particular open-chain or cyclic ammonium compounds such as benzylammonium, diisopropylammonium, triethylammonium or cyclohexylammonium, and complex cations, where appropriate with a metallic central atom such as, for example, iron(III), chromium(III) or cobalt(II) and neutral, cationic or anionic ligands such as, for example, water, ammonia, carbonyl, cyano or nitroso, or oxo cations such as oxovanadium(V) (VO₂⁺) or oxovanadium(IV) (VO²⁺).

8. Sustained release form according to any of Claims 1 to 7, **characterized in that** component (a) comprises a cationic polymer selected from chitosan (poly-D-glucosamine) or chitosan salts (such as, for example, chitosan hydrochloride, acetate, glutamate), poly-L-lysine, basic lectins (glycoproteins, e.g. from extracts such as phytohaemagglutinins), or other basic polypeptides, polysaccharides (such as, for example, hexosamine sugars) or biopolymers of plant, animal or synthetic origin, and any mixtures thereof.

9. Sustained release form according to any of Claims 1 to 8, **characterized in that** the proportion of cationic polymer is from 0.1 to 90% by weight, in particular 5 to 50% by weight, in each case based on the weight of components (a), (b) and (c) in the sustained release form.

10. Sustained release form according to any of Claims 1 to 9, **characterized in that** the α-lipoic acid component is present in proportions of from 0.1 to 99% by weight, in particular in proportions of from 20 to 90% by weight, in each case based on the weight of components (a), (b) and (c) in the sustained release form.

11. Sustained release form according to any of Claims 1 to 10, **characterized in that** the acid component (c) comprises an organic or inorganic Brønstedt acid, in particular acetic acid, hydrochloric acid or glutamic acid.

12. Sustained release form according to any of Claims 1 to 10, **characterized in that** the acid component (c) comprises an organic or inorganic Lewis acid, in particular carbon dioxide, Ca²⁺ or Fe²⁺.

13. Sustained release form according to any of Claims 1 to 10, **characterized in that** the acid component (c) comprises a complex acid, in particular hexaaquoaluminium(III) [Al(H₂O)₆³⁺] or hexacyanoiron(II) acid [H₄(Fe(CN)₆)].

14. Sustained release form according to any of Claims 1 to 10, **characterized in that** the acid component (c) comprises a polymeric acid, in particular polyphosphoric acid (PPA), an isopolyacid such as, for example, heptamolybdic acid (H₆M₇O₂₄), or a heteropolyacid such as, for example, dodecatungstophosphoric acid (H₃[PW₁₂O₄₀]).

15. Sustained release form according to any of Claims 1 to 14, **characterized in that** the acid component (c) is present in proportions of from 0.001 to 80% by weight, in particular in proportions of from 0.1 to 50% by weight and particularly preferably in proportions of from 1.0 to 25% by weight, in each case based on the weight of components (a), (b) and (c) in the sustained release form.

16. Sustained release form according to any of Claims 1 to 15, **characterized in that** it additionally comprises formulation aids such as fillers, lubricants, flow aids, mould release agents, plasticizers, blowing agents, stabilizers, colorants, extenders, binders, disintegrants, wetting agents, glidants or non-stick agents.

17. Sustained release form according to Claim 16, **characterized in that** it comprises as fillers inorganic fillers such as, for example, oxides of magnesium, aluminium, silicon or titanium, microcrystalline cellulose and cellulose powder, starches and derivatives thereof (for example maltodextrins), lactose, mannitol and calcium disphosphate, as lubricants stearates of aluminium and calcium, talc or silicones, as flow aids magnesium stearate, colloidal silica, talc or Aerosil, as plasticizers low molecular weight polyalkylene oxides, low molecular weight organic plasticizers such as glycerol, pentaerythritol, glycerol monoacetate, diacetate or triacetate, propylene glycol, sorbitol or Na diethyl sulphonsuccinate, as colorants azo dyes, (in)organic pigments or natural colouring agents, or other conventional excipients such as sugar (alcohols), polymers, phosphates and surfactants, preferably in respective proportions between 0.02 to 50% by weight, based on the total weight.

18. Sustained release form according to any of Claims 1 to 17, **characterized in that** it is obtainable by
1) mixing component (a) with component (c), preferably in the ratio 1:2 to 1:4 by weight, then adding water to this mixture, and homogenizing the resulting mixture with the α-lipoic acid component (b) in the preferred mixture:component (b) ratio of 1:0.3-0.003 by weight,
2) subjecting the homogenate from 1) to a wet granulation, and drying the granules at temperatures between 5 and 50°C, preferably between 25 and 40°C, and
3) tableting the dry granules.

19. Use of the sustained release form according to any of Claims 1 to 18 for producing a food supplement.

20. Use of the sustained release form according to any of Claims 1 to 18 for producing a medicament.

21. Use of the sustained release form according to any of Claims 1 to 18 for producing a cosmetic.

22. Use according to any of Claims 19 to 21 for producing oral, dermal, parenteral, vaginal or local (topical) administration forms.

23. Use according to any of Claims 19 to 22 for producing gels, semisolid dosage forms or solid solutions or as base thereof.

24. Use according to any of Claims 19 to 23 for improving the absorption of α-lipoic acid and derivatives thereof.

25. Use according to any of Claims 19 to 24 for prolonging the controlled delivery of active ingredient to a period of more than about 8 hours.

26. Use according to any of Claims 19 to 25 for increasing the bioavailability of α-lipoic acid or/and derivatives thereof.

## Revendications

1. Formulation retardatrice qui contient
(a) un ou plusieurs polymères cationiques,
(b) de l'acide α-liponique et/ou un dérivé de celui-ci et
(c) au moins un acide différent de (b).

2. Formulation retardatrice selon la revendication 1 **caractérisée en ce que** le composant (b) contient un acide α-liponique racémique, un acide R-(+)- ou S-(-)-α-liponique énantiomériquement pur ou des mélanges de ceux-ci.

3. Formulation retardatrice selon la revendication 1 **caractérisée en ce que** le composant (b) contient un acide dihydroliponique racémique, un acide (+)-dihydroliponique ou (-)-dihydroliponique énantiomériquement pur ou des mélanges de ceux-ci.

4. Formulation retardatrice selon l'une des revendications 1 à 3 **caractérisée en ce que** l'acide α-liponique ou l'acide dihydroliponique se présente entièrement ou partiellement sous forme de ses sels.

5. Formulation retardatrice selon la revendication 4 **caractérisée en ce que** les sels de l'acide α-liponique ou de l'acide dihydroliponique contiennent des cations du groupe des métaux alcalins ou alcalinoterreux.

6. Formulation retardatrice selon la revendication 4 **caractérisée en ce que** les sels de l'acide α-liponique ou de l'acide dihydroliponique contiennent des cations du groupe du fer, du cuivre, du zinc, du palladium, du vanadium et du sélénium.

7. Formulation retardatrice selon la revendication 4 **caractérisée en ce que** les sels de l'acide α-liponique ou de l'acide dihydroliponique contiennent des cations organiques, en particulier des composés d'ammonium à chaîne ouverte ou cycliques comme le benzylammonium, le diisopropylammonium, le triéthylammonium, le cyclohexylammonium, des cations complexes éventuellement avec des atomes centraux métalliques comme par exemple le fer-III, le chrome-III ou le cobalt-II ou des ligands neutres, cationiques ou anioniques, comme par exemple l'eau, l'ammoniac, le carbonyle, un groupe cyano ou nitroso ou des oxocations comme l'oxovanadium (V) (VO₂⁺) ou l'oxovanadium (IV) (VO²⁺).

8. Formulation retardatrice selon l'une des revendications 1 à 7 **caractérisée en ce que** le composant (a) contient un polymère cationique choisi parmi le chitosane (poly-D-glucosamine) ou les sels de chitosane (comme par exemple le chlorhydrate, l'acétate ou le glutamate de chitosane), la poly-L-lysine, les lectines basiques (glycoprotéines par exemple issues d'extraits comme les phytohémagglutinines) ou d'autres polypeptides, polysaccharides (comme par exemple les glucides d'hexosamine) basiques ou des biopolymères basiques d'origine végétale, animale ou synthétique ainsi que leurs mélanges quelconques.

9. Formulation retardatrice selon l'une des revendications 1 à 8 **caractérisée en ce que** la proportion de polymère cationique est comprise entre 0,1 et 90 % en masse, de préférence comprise entre 5 et 50 % en masse, à chaque fois sur la base de la masse des composants (a), (b), et (c) de la formulation retardatrice.

10. Formulation retardatrice selon l'une des revendications 1 à 9 **caractérisée en ce que** le constituant acide α-liponique est présent dans des proportions comprises entre 0,1 et 99 % en masse, de préférence dans des proportions comprises entre 20 et 90 % en masse, à chaque fois sur la base de la masse des composants (a), (b), et (c) de la formulation retardatrice.

11. Formulation retardatrice selon l'une des revendications 1 à 10 **caractérisée en ce que** le composant acide (c) contient un acide de Brønsted organique ou inorganique, en particulier l'acide acétique, l'acide chlorhydrique ou l'acide glutamique.

12. Formulation retardatrice selon l'une des revendications 1 à 10 **caractérisée en ce que** le composant acide (c) contient un acide de Lewis organique ou inorganique, en particulier le gaz carbonique, le Ca²⁺ ou le Fe²⁺.

13. Formulation retardatrice selon l'une des revendications 1 à 10 **caractérisée en ce que** le composant acide (c) contient un acide complexe, en particulier l'hexa-aquo-aluminium-(III) acide [Al(H₂O)₆³⁺] ou l'hexacyanofer-(II)-acide [H₄(Fe(CN)₆)].

14. Formulation retardatrice selon l'une des revendications 1 à 10 **caractérisée en ce que** le composant acide (c) contient un acide polymère, en particulier l'acide polyphosphorique (PPA), un acide isopoly comme par exemple l'acide d'heptamolybdène (H₆M₇O₂₄) ou un acide hétéropoly comme par exemple l'acide polyphosphorique de tungstène (H₃[PW₁₂O₄₀]).

15. Formulation retardatrice selon l'une des revendications 1 à 14 **caractérisée en ce que** le composant acide (c) est présent dans des proportions comprises entre 0,001 et 80 % en masse, en particulier dans des proportions comprises entre 0,1 et 50 % en masse, et plus particulièrement dans des proportions comprises entre 1,0 et 25 % en masse, à chaque fois sur la base de la masse des composants (a), (b), et (c) de la formulation retardatrice.

16. Formulation retardatrice selon l'une des revendications 1 à 15 **caractérisée en ce qu'**elle contient de plus des auxiliaires de formulation comme des matériaux de charge, des matières lubrifiantes, des auxiliaires de fluidification, des lubrifiants de moule, des émollients, des agents propulseurs, des stabilisateurs, des colorants, des diluants, des liants, des agents de désagrégation (Sprengmittel), des agents mouillants, des agents fluidifiants ou des anti-agglutinants.

17. Formulation retardatrice selon la revendication 16 **caractérisée en ce qu'**elle contient en tant que matériaux de charge des matériaux de charge inorganiques comme par exemple les oxydes de magnésium, d'aluminium, de silicium ou de titane, la cellulose microcristalline et la poudre de cellulose, les amidons et leurs dérivés (par exemple la maltodextrine), le lactose, le mannitol et le diphosphate de calcium, en tant que matières lubrifiantes le stéarate d'aluminium et de calcium, la poudre de talc ou la silicone, en tant qu'auxiliaires de fluidification le stéarate de magnésium, le dioxyde de silicium colloïdal, la poudre de talc ou l'aérosil, en tant qu'émollients les oxydes de polyalkylène de faible poids moléculaire, les émollients organiques de faible poids moléculaire comme la glycérine, le penta-érythrite, le monoacétate, le diacétate ou le triacétate de glycérine, le propylèneglycol, la sorbitol ou le sulfonesuccinate de diéthyle, en tant que colorants les colorants azoïques, les pigments (in)organiques ou les colorants naturels ou d'autres adjuvants habituels comme les glucides (alcools glucidiques), les polymères, les phosphates et les agents tensioactifs, de préférence respectivement dans des proportions comprises entre 0,02 et 50 % en masse sur la base de la masse totale.

18. Formulation retardatrice selon l'une des revendications 1 à 17 **caractérisée en ce qu'**on peut l'obtenir du fait que
1) le composant (a) est mélangé au constituant (c) de préférence selon un rapport de masse compris entre 1:2 et 1:4, puis cette mixture est mélangée avec de l'eau et la mixture obtenue est homogénéisée avec la mixture de composant (b) d'acide α-liponique dans le rapport de masse recommandé : constituant (b) 1 : 0,3 - 0,003,
2) l'homogénat résultant de 1) est soumis à une granulation humide puis le granulat est séché à des températures comprises entre 5 et 50°C, de préférence entre 25 et 40 °C, et
3) le granulat sec est mis en comprimés.

19. Utilisation de la formulation retardatrice selon l'une des revendications 1 à 18 pour fabriquer un complément alimentaire.

20. Utilisation de la formulation retardatrice selon l'une des revendications 1 à 18 pour fabriquer un médicament.

21. Utilisation de la formulation retardatrice selon l'une des revendications 1 à 18 pour fabriquer un cosmétique.

22. Utilisation de la formulation retardatrice selon l'une des revendications 19 à 21 pour fabriquer des formules d'application perorales, cutanées, parentérales, vaginales ou locales (topiques).

23. Utilisation de la formulation retardatrice selon l'une des revendications 19 à 22 pour fabriquer des gels, des formules d'administration semi-solides ou dès solutions solides ou en tant que support pour celles-ci.

24. Utilisation de la formulation retardatrice selon l'une des revendications 19 à 23 pour améliorer la résorption de l'acide α-liponique et de ses dérivés.

25. Utilisation de la formulation retardatrice selon l'une des revendications 19 à 24 pour prolonger l'émission contrôlée d'agent actif afin qu'elle atteigne plus de 8 heures environ.

26. Utilisation de la formulation retardatrice selon l'une des revendications 19 à 25 pour augmenter la biodisponibilité de l'acide α-liponique et/ou de ses dérivés.
